(19) Europäisches Patentamt
European Patent Office
Office européen des brevets

(11) Publication number: 0 254 534 A2

# (12) EUROPEAN PATENT APPLICATION

(21) Application number: 87306445.5

(22) Date of filing: 21.07.87

(51) Int. Cl.4: C 07 H 17/08
C 07 D 313/00,
C 07 D 407/12, A 61 K 31/70,
A 61 K 31/365
//(C07D407/12,313:00,309:00)

(30) Priority: 24.07.86 US 889791 31.12.86 US 948232
14.01.87 US 3080

(43) Date of publication of application:
27.01.88 Bulletin 88/04

(84) Designated Contracting States:
AT BE CH DE ES FR GB GR IT LI LU NL SE

(71) Applicant: Robinson, William S.
1630 Escobita Avenue
Palo Alto California 94306 (US)

(72) Inventor: Robinson, William S.
1630 Escobita Avenue
Palo Alto California 94306 (US)

(74) Representative: Harrison, David Christopher et al
MEWBURN ELLIS & CO 2/3 Cursitor Street
London EC4A 1BQ (GB)

Claims for the following Contracting States: AT + ES + GR.

## (54) Erythromycin derivatives and compositions and use for inhibiting virus replication and disease.

(57) Erythromycin derivatives of the formula

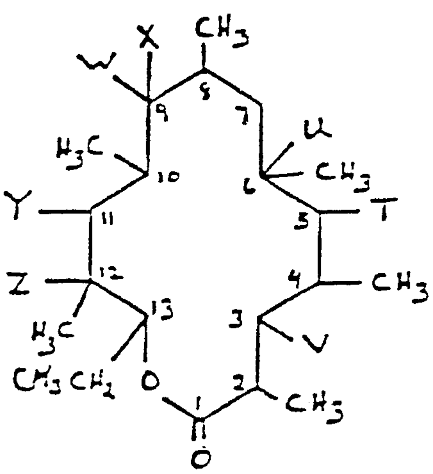

wherein:

T is OH or a pharmaceutically acceptable organic substituent attached to C5 through an oxygen;
V is OH or a pharmaceutically acceptable organic substituent attached to C3 through an oxygen;
U is hydrogen, hydroxy, or alkoxy or acyloxy of from 1 to 10 carbons, or U at C6 and an H at C7 are removed to form a double bond between C6 and C7, or U is taken together with X to define an ether bridge between C6 and C9;
Y is hydrogen, hydroxy, alkoxy or acyloxy of from 1 to 10 carbon atoms, a sulphate or sulfonate group bonded to C11 through an oxygen atom, $-OCH_2SO_2CH_3$, or $-OCH_2SOCH_3$; Y and H at C10 are removed to form a double bond between C10 and C11; or Y and W, when taken together with C9, C10 and C11 of the macrolide ring, represent a 5- to 7-membered heterocyclic ring; and Z, X and W independently represent hydrogen, hydroxy, or acyloxy or alkoxy of 1 to 10 carbon atoms; with the provision that X and W taken together with U as defined above; W can be taken together with Y as defined above; or Z can be taken together with a hydrogen on C13 to represent a double bond; are used in the prevention and treatment of diseases caused by viruses, especially retroviruses and hepadna viruses. Preferred derivatives are oximinoethers or vinyl ethers or have specified substituents in the macrolide or sugar rings.

EP 0 254 534 A2

## Description

ERYTHROMYCIN DERIVATIVES AND COMPOSITIONS AND USE FOR INHIBITING VIRUS REPLICATION AND DISEASE

The subject invention relates to a method of inhibiting replications of viruses in mammals, especially human retroviruses and hepadna viruses, to ameliorate the associated diseases.

Significant progress has been made in preventing and curing bacterial disorders throughout the world. On the other hand, progress against viral disease vectors has been significantly less evident. In underdeveloped countries, viral diseases such as yellow fever and Japanese encephalitis are widespread. Even in developed countries a number of viral disorders exist for which no effective treatment is known once infection has occurred. In the United States the most prevalent viral disorders of this type are caused by herpes simplex type 1 and 2 virus, Vericella-Zoster virus, cytomegalovirus, Epstein-Barr virus, Influenza A and B viruses, respiratory syncitial virus, parainfluenza viruses, rhino viruses, corona viruses, adeno virus, rotavirus, Norwalk virus, hepatitis B virus, Hepatitis A virus, Non-A Non-B hepatitis viruses, papova viruses (JC virus, BK virus, and Papilloma virus), and retroviruses such as HTLV III.

Several human retroviruses are known to be associated with human disease. Human T-lymphotropic virus (HTLV) I and II are associated with human T cell leukemia, and HTLV III (also known as lymphadenopathy associated virus or LAV-1, and human immunodeficiency virus or HIV) and LAV-2 are associated with acquired immunodeficiency syndrome. These retroviruses have a common mechanism of replication and similar biological properties. There is no satisfactory treatment for the diseases associated with these virus infections at present, and the diseases cause serious morbidity and high mortality.

Additionally, a group of DNA viruses exists, known as the hepadnavirus family, having a characteristic of causing infections of the liver and liver disease in their hosts. Included in the known members of the family are hepatitis B virus (HBV) which infects man, woodchuck hepatitis virus (WHB), ground squirrel hepatitis virus (GSHV), and duck hepatitis B virus (DHBV). The members of the hepadna virus family are closely related to each other and share common features of ultrastructure, antigenic structure, molecular structure, mechanism of replication, liver tropism, propensity to cause chronic infection, high concentrations of virus and viral antigen particles in the blood during acute and chronic infections, similar routes of transmission and epidemiologic behavior, and similar spectrum of associated diseases.

HBV-infected individuals with virus in their blood are contagious, and the common routes of transmission to others are by blood, serum, and possibly some other body fluids via parenteral routes, by sexual contacts, and from infected mothers to their newborn or young children. Acute and chronic HBV infections may be associated with acute and chronic hepatitis, viral antigen-antibody (immune) complex mediated diseases, such as glomerulonephritis and polyarteritis, and hepa tocellular carcinoma (HCC). In highly endemic geographic areas of the world, hepatitis B virus commonly infects newborn or young children, whereby chronic infection is established and continues for many years. Early deaths occur in individuals with chronic HBV infection who develop severe hepatitis, cirrhosis, HCC, or polyarteritis.

There is, therefore, an important need to find methods to inhibit or suppress virus replication or to eradicate or terminate the infection in order to eliminate contagiousness; prevent or delay development of disease; and ameliorate, slow progression, or cure disease resulting from infection with viruses.

### DESCRIPTION OF THE RELEVANT LITERATURE

Erythromycin is a well-known, orally effective antibiotic discovered in 1952 and is the subject of many publications. A discussion of erythromycin and its known properties is set forth in the Pharmacological Basis of Therapeutics, 5th Edition, Goodman and Gillman, Eds., MacMillan Publishing Company, New York, 1975, pp. 1224-1227. U.S. Patent Nos. 3,681,325 and 3,979,511 describe oximinoether derivatives of erythromycins, which are reported to be inhibitors of reverse transcriptase from Rous sarcoma in chickens.

### SUMMARY OF THE INVENTION

Erythromycin A and/or B derivatives are employed for prophylactic or therapeutic purposes in the prevention or treatment of virus infections in cells of or from susceptible hosts, especially the prevention and treatment of diseases associated with hepadna and retrovirus infections and the reduction or elimination of contagiousness. Particularly, the compound(s) is administered to cells, typically in a host, in a virus-replication-inhibiting amount prior to or during virus infection, or prior to or during the occurrence of disease associated with the virus infection.

### DESCRIPTION OF SPECIFIC EMBODIMENTS

Patients susceptible to virus infection, especially retrovirus infection or hepadna virus infection, are given prophylactic or therapeutic doses of at least one derivative of erythromycin A or B as identified below, by itself or in combination with other drugs, to prevent virus infection or to suppress or eradicate ongoing virus infections.

The compounds which are employed are derivatives of erythromycin A or B and related macrolide antibiotics. Erythromycin itself has the macrolide ring structure shown below as compound I(a) and I(b).

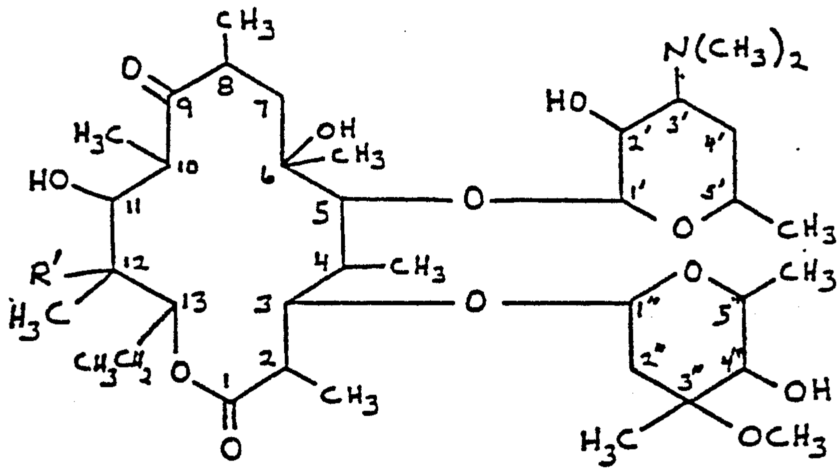

I (a) R' = OH ; Erythromycin A

(b) R' = H ; Erythromycin B

Many compounds of the invention are either erythromycin 9-oximinoethers or 6,9-hemiketal-8,9-dehydroerythromycins (also known as $\Delta^8$-9-desoxo-6,9-oxyerythromycins) using the numbering system shown in the macrolide ring structure above. Preferred embodiments of this aspect of the invention have the following general formula:

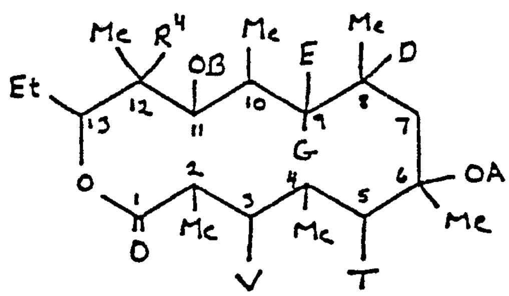

wherein:
Me is methyl;
Et is ethyl;
T is OH or a pharmaceutically acceptable organic substituent attached to C5 through an oxygen (especially a sugar residue);
V is OH or a pharmaceutically acceptable organic substituent attached to C3 through an oxygen (especially a sugar residue);
A is hydrogen or acyl (preferably alkanoyl or other carboxylic acid derivatives of from 1 to 20 carbons which may be substituted, including carboxylic acid derivatives having aliphatic, alicyclic, or aromatic (including heteroaromatic) organic moieties attached to -CO-, or a combination thereof), or OA at C6 and an H at C7 are removed to form a double bond between C6 and C7, or A is taken together with G to define a bond, the bond forming a vinyl ether bridge between C6 and C9;
B is hydrogen, acyl (preferably alkanoyl as defined for A), a sulfate or sulfonate group (including an alkyl sulfate or sulfonate containing from 1 to 6 carbon atoms), $CH_2SO_2CH_3$, or $CH_2SOCH_3$; or OB and an H at C10 are removed to form a double bond between C10 and C11;
D is hydrogen or is taken together with E to define a double bond;
E is taken together with D to form a double bond, E is taken together with G to define an oximinoether in which the oxygen is substituted with R, or E and G are taken together to represent a diazo group or a diazo group substituted with pharmaceutically acceptable organic substituent;
R is a pharmaceutically acceptable organic substituent; and
$R^4$ is hydrogen, hydroxyl, or hydroxyl substituted with a pharmaceutically acceptable organic substituent.

When T and V represent sugar residues, the sugar residues preferably result from an acetal formation between an aldose and an hydroxyl group on the macrolide ring at the positions of the V and T substituents (C3 and C5, respectively). The sugar is preferably present in a pyranose (6-membered ring) form. When T represents a sugar, the sugar is preferably an amino sugar, especially an amino sugar with the amino or substituted amino group present at the 3′ position. Preferred sugars (for both positions) also contain fewer than one hydroxyl group per non-carbonyl carbon (e.g., fewer than five hydroxyl groups in a 6-carbon aldose). Especially preferred for T is desosamine and for V, cladinose. Substituents can be present on the sugar residue or residues.

The phrase "pharmaceutically acceptable organic substituent" as used in this specification (whether for this or later general formulas) is intended to encompass stable organic substituents that do not prevent the compound under consideration from inhibiting viral replication. However, it should be recognized that inhibition of viral replication need not be complete. Amelioration of viral diseases occurs in many disorders when the rate of viral replication is merely reduced. Reducing the rate of viral replication allows the natural immune system to function more effectively.

Pharmaceutically acceptable organic substituents (other than sugars) are preferably no larger in bulk than a benzyl or benzoic acid substituent that itself is substituted with C1-C4 alkyl substituents. Preferred substituents on aromatic groups include halo, C1-C4 alkyl or alkanoyl, C1-C4 alkoxy or alkanoyloxy, hydroxy, carboxy (and alkylethers), amino, alkyl or dialkyl amino, and nitro groups. Preferred substituents on non-aromatic carbons include all of those preferred for aromatic substituents except nitro. Preferred substituents on a heteroatom (e.g., oxygen or nitrogen) include substituents in which attachment is through an organic carbon. When undefined substituents are present on a specifically designated principal substituent (for example, when R is a "substituted benzyl group"), they are preferably limited to one or two (e.g., 2,4-dichlorobenzyl). Preferred heterocyclic rings, both aromatic and non-aromatic, include furan, thiophene, pyrrole, pyrazole, imidazole, triazole, oxazole, thiazole, isothiazole, pyran, pyrone, dioxin, pyridine, pyridazine, pyrimidine, pyrazine, piperazine, triazine, oxazine, and morpholine. Preferred pharmaceutically acceptable organic substituents contain no more than one organic ring, no more than one substituent or functional group per carbon atom (except for halogenated hydrocarbons) and more preferably no more than one substituent or functional group per two carbon atoms, and 15 or fewer, more preferably 10 or fewer, atoms other than hydrogen.

A number of preferred compounds of the invention have the general formula set forth above in which the different parts of the formula have the following meanings:

Me is methyl;

Et is ethyl;

T is OH, alkoxy of from 1 to 6 carbons, acyloxy of from 1 to 22 carbons (especially 1 to 3 carbons and $C_{10}$-$C_{22}$ naturally occurring fatty acid acyl groups), 2-methyl-4-($R^1R^2N$)-5-$R^3$-pyranyl-6-oxy, 2-methyl-3,4-epoxy-5-$R^3$-pyranyl-6-oxy, or 2-methyl-3,4-dehydro-5-$R^3$-pyranyl-6-oxy, wherein $R^1$ represents hydrogen or methyl, $R^2$ represents hydrogen, methyl, benzyl, or benzyl substituted with a pharmaceutically acceptable organic substituent, especially a halo, $C_1$-$C_4$ alkyl or alkanoyl, hydroxy, carboxy, amino, alkyl or dialkyl amino, or nitro group, and $R^3$ is hydroxy or a pharmacologically acceptable alkyl, alkoxy, or acyloxy of from 1 to 10, preferably 1 to 6, carbon atoms, e.g. formyloxy, acetoxy, propionoxy, or ethyl succinoxy; acyloxy groups for $R^3$ include groups in which the acyl moiety is within the definition of A above and especially includes nicotinyl;

V is 2,4-dimethyl-3-$R^3$-4-methoxypyranyl-6-oxy, hydroxy, alkoxy of from 1 to 6 carbons, acyloxy of from 1 to 22 carbons (especially 1 to 3 carbons and $C_{10}$-$C_{22}$ natually occurring fatty acid acyl groups), sulfonate, or alkyl-substituted sulfonate;

B is hydrogen, alyloxy, $CH_2SO_2CH_3$, or $CH_2SOCH_3$;

D is hydrogen or is taken together with E to define a double bond;

E is taken together with D to form a double bond or taken together with G to define an oximinoether where the oxygen is substituted with R; E and G taken together also can represent a diazo substituent, which may be substituted by a pharmaceutically acceptable organic substituent, especially R;

A is hydrogen or acyl of from 1 to 3 carbons, or OA at C6 and C7 are removed to form a double bond between C6 and an H at C7, or A is taken together with G to define a bond, defining a vinyl ether bridge;

$R^4$ is hydrogen or hydroxyl; and

R is an organic substituent of from 1 to 20 carbon atoms, particularly a hydrocarbyl or substituted hydrocarbyl groups which can be aliphatic, alicyclic, aromatic (including heteroaromatic) or a combination thereof and further can be aliphatically saturated or unsaturated, particularly saturated. R more particularly is alkyl of from 1 to 3 carbon atoms, preferably 1 to 2 carbon atoms (optionally substituted with one or more alkoxy groups, e.g., methoxyethoxymethyl); cycloalkyl with 5 or 6 carbon atoms in the cycloalkyl ring; phenyl; benzyl; benzoyl; or substituted derivatives thereof, particularly substituted benzyl where the substituents are halo (particularly chloro, more particularly mono or dichloro bonded to an aryl carbon atom). Illustrative R groups include methyl, ethyl, propyl, cyclohexyl, phenyl, benzyl, 2,4-dichlorobenzyl, methoxymethyl, benzoyl, and alkyl- or halo-substituted benzoyl. Hydrocarbyl refers to an organic group composed solely of hydrocarbon and carbon and can be aliphatic, aromatic, alicyclic or combinations thereof and can be saturated or unsaturated; substituted hydrocarbyl intends the presence of heteroatoms (especially O, N, S, P, and halogens as well as metal salts, especially alkali metal salts, of acids) and includes heterocyclic groups.

Other suitable groups represented by E and G taken together include the following:

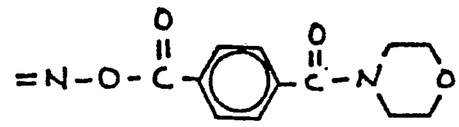

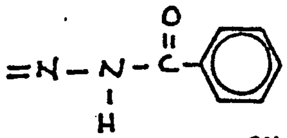

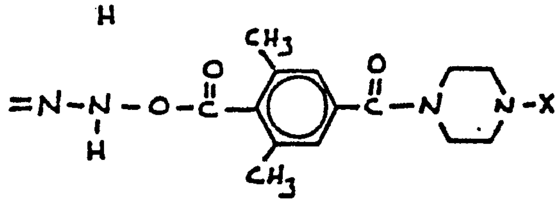

X = $C_1$-$C_4$ alkyl or hydroxyalkyl

EMI PA=9 FR=1 HE=65 WI=130 TI=CHE

Particularly preferred compounds are those in which all of the substituents in the general formula set forth above are identical to those present in natural erythromycins with the provisos that $R^4$ can represent either hydroxy or hydrogen (therefore defining erythromycin A and B derivatives, respectively) and R can represent any of the substituents set forth in the immediately preceding two paragraphs. At least one difference from erythromycin A and B is required.

Examples of specific compounds of the invention include 9-0-methyloximinyl erythromycin A (including the isolated 9(Z) and 9(E) isomers); 9-0-(2,4-dichlorobenzyl)oximinyl erythromycin A; 9-0-isopropyloximinyl erythromycin B; 9-0-phenyloximinyl erythromycin B; 9-0-cyclohexyloximinyl erythronolide A; 9-0-(2-methoxyethoxy)methyloximinyl erythromycin A; 9-0-hexadecyloximinyl erythromycin A; des-dimethylamino-9-0-methyloximinyl erythromycin A; 9-0-methyloximinyl erythronolide A; 9-0-methyloximinyl erythronolide A 2′,4″-dinicotinate; 3′-de(dimethylamino)-3′,4′-didehydro-9-0-methyloximinyl erythronolide A 2′4″-dinicotinate; 9-0-benzyloxyminyl erythronolide A N-oxide; 9-0-isopentyloximinyl erythronolide A; and 9-0-methyloximinyl erythronolide A 4′-monoacetate.

A second group of preferred compounds of the invention have the following second general formula:

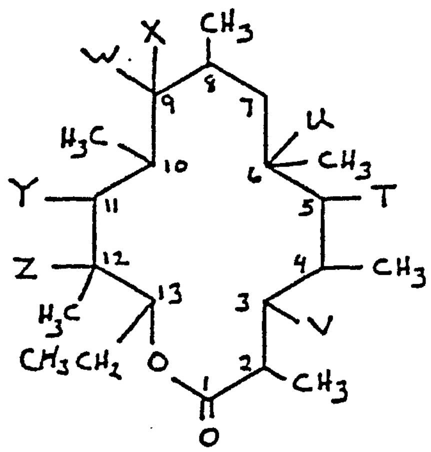

wherein:

T is OH or a pharmaceutically acceptable organic substituent attached to C5 through an oxygen (especially a sugar residue);

V is OH or a pharmaceutically acceptable organic substituent attached to C3 through an oxygen (especially a sugar residue);

U is hydrogen, hydroxy, or alkoxy or acyloxy (preferably alkanoyl) of from 1 to 10 carbons, or U at C6 and an H at C7 are removed to form a double bond between C6 and C7, or U is taken together with X to define an ether bridge between C6 and C9;

Y is hydrogen, hydroxy, alkoxy or acyloxy (preferably alkanoyl) of from 1 to 10 carbon atoms, a sulphate or sulfonate group (including an alkyl sulphate or sulfonate containing from 1 to 6 carbon atoms) bonded to C11 through an oxygen atom, $-OCH_2SO_2CH_3$, or $-OCH_2SOCH_3$; Y and an H at C10 are removed to form a double bond between C10 and C11; or Y and W, when taken together with C9, C10, and C11 of the macrolide ring,

represent a 5- to 7-membered heterocyclic ring, especially a 6-membered 1,3-dioxa ring optionally containing boron at the 2-position; and

Z, X, and W independently represent hydrogen, hydroxy, acyloxy (preferably alkanoyl) of from 1 to 10 carbon atoms, or alkoxy (preferably 1 to 6 carbon atoms); with the provision that X and W taken together can represent =O or =S; X can be taken together with U as defined above; W can be taken together with Y as defined above; or Z can be taken together with a hydrogen on C13 to represent a double bond.

When T and V represent sugar residues, the sugar residues preferably result from an acetal formation between an aldose and an hydroxyl group on the macrolide ring at the positions of the V and T substituents (C3 and C5, respectively). The sugar is preferably present in a pyranose (6-membered ring) form. When T represents a sugar, the sugar is preferably an amino sugar, especially an amino sugar with the amino or substituted amino group present at the 3′ position. Preferred sugars (for both positions) also contain fewer than one hydroxyl group per non-carbonyl carbon (e.g., fewer than five hydroxyl groups in a 6-carbon aldose). Especially preferred for T is desosamine and for V, cladinose. Substituents can be present on the sugar residue or residues as described for the first general formula.

A number of preferred compounds of the invention have the second general formula set forth above in which the different parts of the formula have the following meanings:

T is OH, alkoxy of from 1 to 6 carbons, acyloxy of from 1 to 22 carbons (especially 1 to 3 carbons and $C_{10}$-$C_{22}$ naturally occurring fatty acid acyl groups), 2-methyl-4-($R^1R^2N$)-5-$R^3$-pyranyl-6-oxy, 2-methyl-3,4-epoxy-5-$R^3$-pyranyl-6-oxy, or 2-methyl-3,4-dehydro-5-$R^3$-pyranyl-6-oxy, wherein $R^1$ represents hydrogen or methyl, $R^2$ represents hydrogen, methyl, benzyl, or benzyl substituted with a pharmaceutically acceptable organic substituent, especially a halo, $C_1$-$C_4$ alkyl or alkanoyl, hydroxy, carboxy, amino, alkyl or dialkyl amino, or nitro group, and $R^3$ is hydroxy or a pharmacologically acceptable alkyl, alkoxy, or acyloxy of from 1 to 10, preferably 1 to 6, carbon atoms, e.g. formyloxy, acetoxy, propionoxy, ethyl succinoxy, 3,4-dichloro-benzyloxy, or nicotinyloxy;

V is 2,4-dimethyl-3-$R^3$-4-methoxypyranyl-6-oxy, hydroxy, alkoxy of from 1 to 6 carbons, acyloxy of from 1 to 22 carbons (especially 1 to 3 carbons and $C_{10}$-$C_{22}$ naturally occurring fatty acid acyl groups), sulfonate, or alkyl-substituted sulfonate;

Y is hydrogen, hydroxy, or acyloxy, or Y and W are taken together with C9, C10, and C11 to represent a 6-membered 1,3-dioxa ring or a 6-membered 1,3-dioxa-2-bora ring substituted with a phenyl group on the boron atom; and

Z, X, and W have the meanings defined above.

Compounds of this second aspect of the invention differ from erythromycin A or B (whichever is more closely related) by at least one substituent represented by T, U, V, W, X, Y, Z, or a combination thereof. Preferred compounds differ at not more than 4, more preferably not more than 3, and most preferably not more than 2 of these locations. When T or V represents a sugar other than desosamine or cladinose, respectively, 4 or fewer, more preferably 3 or fewer, and most preferably 2 or fewer functional groups are different in any one sugar.

The following are examples of specific compounds in which the indicated functional groups are different from natural erythromycin A or B: erythromycin A N-oxide (differing in the amino group on the desosamine sugar); $\Delta^{6,10}$-6,11-dideoxyerythronolide diacetate; erythromycin B 11-methylthiomethyl ether 2′-acetate (with an acetate ester formed at the hydroxy group of the desosamine sugar residue); $\Delta^6$-6-deoxy-11-0-acetyl erythromycin B 2′-acetate; erythromycin B with a 2-methyl-3,4-epoxy-5-acetoxy-6-pyranyl-oxy substituent in place of the desosamine sugar residue; erythromycin B with a 2-methyl-3,4-dehydro-5-acetoxy-pyranyl-6-oxy substituent in place of the desosamine sugar residue; erythromycin B with a 2-methyl-3,4-dehydro-5-acetoxy-pyranyl-6-oxy substituent in place of the desosamine sugar residue and a 2,4-dimethyl-3-acetoxy-4-methoxypyranyl-6-oxy substituent in place of the cladinose sugar residue; and erythromycin B in which the position corresponding to X of the second general formula is a hydrogen and Y and W represent $-O-CH_2-O-$. It should be noted that sugar residues when numbered as pyrans with the ring oxygen being position 1 have different numerical values to indicate substituent positions as compared to the erythromycin system using primes and double-primes.

These compounds are all erythromycins having at least one pharmaceutically acceptable organic substituent selected from macrolide and amino sugar double bonds and epoxides; heterocyclic rings fused to the macrolide ring through positions C9 and C11 (generally including the oxygens of the two hydroxyl or oxo groups present at these locations); and alkyl, acyl; thioalkyl, sulfate, and sulfonate derivatives of hydroxy groups present in natural erythromycins and erythronolides.

When T and V are both hydroxyl groups, the resulting compound is usually called erythronolide rather than erythromycin. However, this compound and similar macrolide compounds in which the substituents have the meaning set forth above are considered to be within the scope of the present invention and to be encompassed by the phrases "erythromycin derivative", "erythromycins", or "compound" and similar phrases as used herein to refer to compounds of the invention.

Pharmaceutically acceptable salts are intended to be identified and encompassed by reference to compounds in their non-salt forms. Various salts of the compounds can be made including stearate or other $C_2$-$C_{20}$ fatty acids, doedecyl and other fatty sulfates, phosphate, hydrochloride, lactobionate, or gluconate. Quaternary amine salts including those formed from amino substituents forming part of the compounds of the invention are also included (especially trimethylamino-sugars).

The oximinyl derivatives or erythromycins can be prepared by the process described in U.S. Patent No. 3,681,326, issued August 1, 1972. The vinyl ether can be obtained by warming the oximino ether in an organic solvent under mildly acidic conditions. All of the compounds can be produced semi-synthetically from natural erythromycins by modifications of organic functional groups, such as hydroxy, acetal, and carbonyl groups, using standard techniques of organic chemistry.

The subject drugs may be formulated in conventional ways employing a physiologically or pharmacologically acceptable carrier. Such carriers include sterilized water, where the drug may be suspended (optionally employing a detergent or emulsifier) or dissolved in phosphate buffered saline, dimethyl sulfoxide (DMSO), ethylene glycol, alcohol, etc. The drug may be formulated as a tablet, capsule, or the like, being encapsulated in accordance with conventional ways.

Compounds of the present invention can be formulated and utilized in the same manner as other erythromycin derivatives. Formulations, dosages, techniques usable for oral administration, and the like for erythromycin and related compounds can be found in the Pharmacological Basis of Therapeutics (cited above), pp. 1224-1227. Formulation and administration is therefore within the skill of those skilled in the art to which this invention pertains. However, the following comments are provided for initial guidance.

The concentration of the drug in a particular formulation will vary depending upon the formulation, the presence or absence of other drugs, the manner of administration, the carrier, and the like. Typical unit dosages will fall in a range from 10 to 500 mg of active ingredient. Orally administratable tablets and capsules containing from 5 to 95% active ingredient are suitable. Parenteral compositions containing 10 to 100 mg/ml can readily be prepared. Sterile dry powders for use as intravenous injection solutions upon mixing with physiological saline are likewise suitable.

The dosage of the subject compounds will generally be at least about 1.0 and not more than about 200 mg per kg of body weight per day in single or multiple doses, usually from about 10 to 100 mg per kg of body weight. The treatment courses can be given for a day, a few days, weeks, months or years, depending upon the effectiveness of the course of treatment, or the refractory nature of the disease.

Erythromycins are known to concentrate in the liver of the host to which they are administered. Accordingly, the compounds of the invention are expected to be particularly useful in the treatment of viral diseases of the liver, such as hepatitis.

Although the compounds of the invention can be utilized in the treatment of animals, particularly mammals, treatment of humans is preferred. The drugs are utilized in treating the diseases and types of viruses discussed in the Background section of the specification. Oximino ethers and related hemiketals and ketals are particularly preferred for retroviruses and hepadna viruses, although compounds of the second general formula can also be used for this purpose.

The drugs either by themselves or in combination may be administered subcutaneously, intradermally, orally, parenterally, intraperitoneally, intravascularly, etc. The particular manner of administration will be selected in order to insure that the drug(s) are able to be directed to the site of viral invasion in an effective dosage (i.e., directed to the susceptible cells).

The subject compositions can be administered in conjunction with, either in the same formulation or in a separate formulation, other drugs which act in cooperation with the drugs of the subject invention or may be employed to provide for various supportive prophylaxis or therapeutic capabilities, such as the prevention, amelioration, or therapy of the diseases for which a retrovirus is the etiologic agent in whole or in part. These agents may include such non-proteinaceous drugs as adenine arabinoside (araA), rifamycin and derivatives thereof, ribavirin, acyclovir and related compounds, and azidothymidine or peptide drugs such as interferon (α, β, or γ), interleukins, e.g., IL-2, tumor necrosis factor, etc.

For adjunctive drugs, the dosage will vary with the intended use; for araA the dosage will be about 5 to 15 mg/kg-day, for the proteins (biologicals) about $10^5$-$10^7$ U/day or the equivalent.

The following examples are offered by illustration and not by way of limitation.

EXAMPLE 1

A sample of 9-0-methyloximinyl erythromycin A (EMO) was tested for capacity to inhibit growth of HTLV III in tissue culture. A T-cell line (VB) was infected with HTLV III and infected cells were incubated with 0, 1, 5, 10 and 20 μgm EMO per ml of tissue culture medium. Culture medium was recovered after 0, 1, 2, 3, and 4 days of incubation and assayed for HTLV III production by reverse transcriptase and viral antigen (P-24) testing by ELISA. Table 1 shows that production of viral antigen and virus-particle-associated reverse-transcriptase activity were significantly reduced.

Table 1

| EMO Concentration (µg/ml) | % Reduction | |
|---|---|---|
| | Reverse Transcriptase | P-24 |
| 0 | 0 | 0 |
| 1 | 10% | 8% |
| 5 | 50% | 45% |
| 10 | 73% | 69% |
| 20 | 82% | 86% |

It is evident from the above results that the subject erythromycin derivatives are effective in inhibiting the replication of human retrovirus in cells in culture. This indicates that compounds of the invention can be used to prevent or inhibit human retrovirus infections in vivo. Low dosages may be employed with compounds which have been proven to be safely administered to humans. The compounds can be administered in a variety of ways, such as orally, so as to be convenient and provide for a high degree of protection both during and subsequent to administration of the drug.

As evidenced by the above results, the subject therapy can be used in the treatment of acute and chronic retrovirus infections to inhibit or suppress virus replication or to eradicate or terminate the infection in order to (1) reduce or eliminate contagiousness, (2) prevent or delay the development of disease when treatment is used in infected individuals before they have developed disease, and (3) ameliorate, slow progression of or cure disease resulting from infection.

EXAMPLE 2

A sample of 9-0-methyloximinyl erythromycin A (EMO) in solution at a concentration of 100 mg/ml in dimethyl sulfoxide (DMSO) was administered subcutaneously at a dose of 15 mg/kg/day for 14 days to a group of 10-week old Pekin ducks chronically infected with DHBV (experimentally infected as described by Marion et al., "Liver Disease Associated with Duck Hepatitis B Virus Infection of Domestic Ducks", Proc. Natl. Acad. Sci. USA (1984) 81:898). Another group of 10-week old Pekin ducks were used as a control and treated with the same dose of DMSO alone. In vivo replication of virus was assessed by measuring levels of DHBV virus in the serum of infected animals before, during, and after treatment. Virus levels in serum were determined by quantitating the DNA of the virus by slot blot hybridization. Ten µl of each serum was made up to 0.1 M with NaOH to disrupt virus and denature the viral DNA. The samples were spotted on nylon membrane which binds single stranded DNA. The filter-bound viral DNA was detected by hybridization with a DHBV [$^{32}P$]- DNA probe followed by autoradiography. The quantity of viral DNA in each serum sample, expressed as picograms of viral DNA/ml serum, was determined by comparing densitometer tracings of the exposed area of film overlying each serum spot on the nylon membrane with a standard curve derived from densitometer tracings for known amounts of DHBV DNA similarly spotted on the same nylon membrane.

The results obtained show that at 0 days the picograms of DHPV DNA/ml serum was at 900, reaching a maximum of 1500 by day 8 and dropping to below the level of detection by day 12. EMO was administered beginning on day 8 for 14 days at the rate of 15 mg/kg/day. The low level was retained until day 22 (when EMO treatment was discontinued) thereafter the viral level began to climb and was followed to day 32, where the level reached about 650 picograms DHPV DNA/ml serum.

Table 2 summarizes the results of treatment of four animals with erythromycin A 9-0-methyloxime (EMO) in DMSO and four animals with DMSO alone as determined on day 22 after termination of administration of the drug after 14 days. Virus was similarly found to be suppressed by EMO treatment in each animal and not by DMSO.

Table 2

| Animal No. | Treatment | % Reduction in DHBV DNA In Serum After 14 Days Treatment |
|---|---|---|
| 1 | EMO (in DMSO) 15 mg/kgm/day | 100 |
| 2 | EMO (in DMSO) 15 mg/kgm/day | 100 |
| 3 | EMO (in DMSO) 15 mg/kgm/day | 96 |
| 4 | EMO (in DMSO) 15 mg/kgm/day | 94 |
| 5 | DMSO 0.5 ml per day | 0 |
| 6 | DMSO 0.5 ml per day | 0 |
| 7 | DMSO 0.5 ml per day | 0 |
| 8 | DMSO 0.5 ml per day | 0 |

EXAMPLE 3

A number of examples of synthetic techniques are set forth to show synthesis of various compounds of the invention.

Preparation of erythromycin A 9-0-methyloxime and separation of the 9(Z)- and 9(E)-isomers

Erythromycin A (86.84 g) was added to a 3 liter three-neck round bottom flask followed by 600 ml of methanol. To this clear solution at room temp/$N_2$ was added 39.2 g of $CH_3ONH_2$ HCl. After stirring for 10 minutes, 32.86 ml of $Et_3N$ was added to the clear solution at room temp/$N_2$. After stirring at room temp/$N_2$ for 20 hours, the reaction was completed. The mixture was cooled with an ice-water bath, and 1 liter of 10 % $NH_4OH$ aqueous solution was slowly added. The white precipitate was collected by filtration, washed with water (3 x 100 ml), and dried in air overnight. The solid material was dissolved in 1.6 liters of $CH_2Cl_2$, and a trace amount of insoluble material removed by filtration. The $CH_2Cl_2$ solution was washed with water (4 x 350 ml) and dried with 45 g of anhydrous $Na_2SO_4$ granules. After filtration, concentration of the clear solution to a volume of 200 ml generated a large amount of white crystalline material. To the white suspension was added 200 ml of absolute ether, and the resulting suspension stirred at room temperature for 5 minutes. The loose and nice crystals were collected, washed with trace amounts of ether, and dried in air overnight to yield 34.7 gm of desired product.

Since its HPLC showed some impurity, further recrystallization was required. The impure product (34 g) was dissolved in 340 ml of $CH_2Cl_2$ and diluted with an equal amount of absolute ether (340 ml). Concentration of the clear solution to a volume of 300 ml generated a large amount of white crystals, which were collected and dried at room temp/0.1 mm Hg overnight to afford 19.0 g of the title product, mp 127-131°C. IR, NMR, MS, and elementary analysis data were in agreement with the structure of the title product.

Separation of the two 9-methyloxime isomers can be achieved by preparative HPLC on C-18 column with the solvent system of $CH_3OH$/0.1 M $(NH_4)HCO_3$, pH 7.0, ratio 85/15. Starting with 210 mg of the mixture, HPLC separation gives firstly 11 mg of the 9(Z)-methyloxime, mp 120-122°C, IR(KBr): 3450, 1735, 1460, 1380, 1167, 1050 and 1010 $cm^{-1}$ NMR ($CDCl_3$): 0.84 (t, 3H, $CH_3$ or

ethyl group), 2.32 (s, 6H, 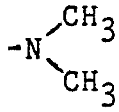 ), 3.32 (s, 3H, 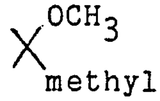 ),

3.82 (s, 3H, $=N\text{-}OCH_3$) ppm. Mass spectrum: MH+ at m/e 763 (theory 763). Further elution obtains 140 mg of the 9(E)-methyloxime, mp 127-130°C, IR (KBr): 3450, 1737, 1460, 1380, 1170, 1050 and 1010 $cm^{-1}$. NMR ($CDCl_3$):δ 0.84 (t, 3H, $CH_3$ of ethyl group), 2.32

(s, 6H, 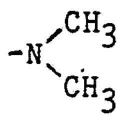 ),

3.32 (s, 3H, 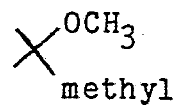 ),

3.85 (s, 3H, $=NOCH_3$) ppm.
Mass spectrum: MH+ at m/e at 763 (theory 763).

Preparation of erythromycin A 9-[0-[(3,4-dichlorophenyl)methyl]oxime]

Erythromycin A (58.64 g) was added to a 3 liter three-neck round bottom flask followed by 800 ml of methanol. To this clear solution at room temp./$N_2$ was added 55 gm of (3,4-dichlorophenyl)methoxyamine hydrochloride (Ref.: Bull. Acad. Pal. Sci., Ser. Sci. Chem. (1974) 22(3):195-199) and 12.65 g of $Et_3N$. Stirring for 48 hours completed the reaction. The mixture was cooled with an ice-water bath, after which 1 liter of 10% $NH_4OH$ aqueous solution was added. Collection of the white precipitate by filtration was followed by washing with water (3 x 100 ml) and drying in air over night. The solid material was dissolved in 1.5 liter of $CH_2Cl_2$, washed with water (4 x 300 ml), and dried over anhydrous $MgSO_4$. After filtration, concentration of the filtrate to about 80 ml generated a large amount of white crystalline material. To this white suspension, 200 ml of absolute ether was added and stirred at room temperature for 1/2 hour. The crystals were collected and recrystallized with $CH_2Cl_2$/ether = 1:1 to afford 23.7 g of the title product, mp 107-109°C. IR, NMR, and MS data were in agreement with the structure of the title product.

Preparation of Roxithromycin (erythromycin A 9-[0-[(2-methoxyethoxy)methyl]oxime])

The title compound can be prepared according to the procedure published in U.S. Patent 4,349,545 (September 14, 1982), mp 110-119°C. IR, NMR, and MS data were in agreement with the structure of the title product.

Preparation of Erythromycin 9 (0-hexadecyloxime)

Erythromycin 9-oxime (39.3 g) was dissolved in 240 ml of dimethyl formamide. To this solution, 36.6 g of hexadecyl bromide and 13.8 g of $K_2CO_3$ were added. After stirring for four days, the mixture was filtered and the filtrate evaporated. The residue was dissolved in 100 ml of methanol and diluted with 1.3 liter of 10% $NH_4OH$ solution. The white precipitate was collected, dissolved in 1.25 liters of methylene chloride, and washed with water. Evaporation of the solvent and HPLC purification of the crude product (C-18 column, solvent system: acetonitrile/0.01 M $NH_4HPO_4$ pH 3.5 buffer = 80/20) gave 41.5 g of the title product as a solid, mp 86-94°C. IR, NMR, and MS data were in agreement with the structure of the title product.

Preparation of des-dimethylaminoerythromycin A 9-0-methyloxime

Erythromycin A 9-methyloxime N-oxide (2.0 g; prepared from erythromycin A 9-methyloxime according to the conversion technique described in J. Am. Chem. Soc. (1954) 76:3121) was pyrolyzed at 145-155°C under reduced pressure (0.1 mm Hg) for 3 hours. Chromatography of the pyrolyzed product on silica gel with 1% methanol in methylene chloride gave 0.28 g of the title product as a solid, mp 127-129°C. IR, NMR, and MS data were in agreement with the structure of the title product.

Preparation of erythronolide A 9-(0-methyloxime)

Des-dimethylamino-erythromycin A 9-methyloxime (0.9g) was dissolved in 70 ml of 3% HCl in methanol. After the solution was stirred for 20 hours at room temperature, the solvent was evaporated and the residue dissolved in 200 ml of ethyl acetate. The ethyl acetate solution was washed with saturated sodium bicarbonate solution and brine, and the resulting organic phase was dried. The solvent was evaporated and the crude product chromatographed on silica gel. Elution with 2% methanol in methylene chloride afforded that title product as a solid, mp 98-101°C. IR, NMR, and MS data were in agreement with the structure of the title product.

Preparation of erythromycin A 9-0-methyloxime dinicotinate

Erythromycin A 9-(0-methyloxime) (1.0 g) was dissolved in 10 ml of pyridine. To this clear solution, 1.0 g of nicotinyl chloride•HCl was added. Stirring at room temperature for 20 hours and evaporation of the pyridine under reduced pressure gave a yellow foam, which was dissolved in 200 ml of methylene chloride, washed with

water, and dried. The solvent was evaporated and the residue chromatographed on silica gel. Elution with 5% methanol in methylene chloride afforded 0.15 g of the title product as a foam, mp 148-152°C. IR, NMR, and MS data were in agreement with the structure of the title product.

Preparation of 3′-de(dimethylamino)-3′,4′-didehydroerythromycin 9-(0-methyloxime) 2′,4″-dinicotinate

Des-dimethylamino-erythromycin A 9-methyloxime (718 mg) was dissolved in 4 ml of pyridine. To the solution, 1.0 g of nicotinyl chloride•HCl was added. Stirring at room temperature for two days and evaporation of the solvent under reduced pressure gave a residue which was purified by silica gel chromatography. Elution with 2% methanol in methylene chloride afforded 350 mg of the title product as a solid, mp 120-130°C. IR, NMR, and MS data were in agreement with the structure of the title product.

Erythromycin 9-(0-benzyloxime) N-oxide

Erythromycin 9-(0-benzyloxime) (2.0 g) was dissolved in 70 ml of methanol and 30 ml of water. To this solution, 12 ml of 30% aqueous hydrogen peroxide solution was added. The mixture was stirred at room temperature under nitrogen for 24 hours, all methanol was evaporated, and the aqueous residue was extracted with $CH_2Cl_2$. The $CH_2Cl_2$ extract was washed with water (2 x 100 ml) and dried over $MgSO_4$. The solvent was evaporated and the residue crystallized with $CH_3OH$-ether mixture to afford 1.26 g of the title product as a white solid, mp 175-177°C. IR, NMR, and MS data were in agreement with the structure of the title product.

Preparation of erythromycin A 9-0-isopentyloxime

At room temperature under nitrogen, 90.0 g of erythromycin and 56.0 g of i-$C_5H_{11}ONH_2$•HCl was dissolved in 650 ml of $CH_3OH$. To the solution, 24.7 g of triethylamine was added and stirred for 24 hours. At 0°C, the mixture was treated with 1 liter of 10% $NH_4OH$ aqueous solution. The white precipitate was collected, rinsed with water (6 x 200 ml), and dried in air overnight. The solid was dissolved in 2.5 liters of $CH_2Cl_2$, washed with water (3 x 1 liter) and dried with $Na_2SO_4$ granules. Evaporation of the $CH_2Cl_2$ solution gave a white solid, which was recrystallized initially with a mixture of $CH_2Cl_2$/ether/pentane (3:13:15). Further recrystallization of the solid material with a mixture of ether/$CH_2Cl_2$/pentane (10:3:15) afforded 25.6 gm of the title product (Wy-49,112), mp 122-125°C. IR, NMR, MS, and elementary analysis data were in agreement with the structure of the title product.

Preparation of erythromycin A 9-methyloxime 4′-monoacetate

Erythromycin A 9-methyloxime (1.0 g) was dissolved in 10 ml of pyridine. To this clear solution, 2 ml of acetic anhydride was added. After stirring at room temp/$N_2$ for 28 hours, 25 ml of water was added, and the resulting mixture was extracted with $CHCl_3$ (4 x 35 ml). The $CHCl_3$ extract was washed with saturated $NaHCO_3$ (3 x 15 ml), then with water (3 x 15 ml), and dried with $MgSO_4$. Evaporation of the solvent and crystallization of the residue with ether/pentane (1:5) mixture gave 750 mg of the impure solid.

The solid (210 mg) was dissolved in 10 ml of aqueous methanol ($H_2O$:$CH_3OH$ = 1:4) and stirred at room temp/$N_2$ for 24 hours. The mixture was diluted with 25 ml of water, extracted with $CHCl_3$ (3 x 35 ml), and the extract dried with $MgSO_4$. Evaporation of the $CHCl_3$ solution afforded 130 mg of the title product (Wy-48,138), mp 125-130°C. IR, NMR, and MS data were in agreement with the structure of the title product.

All publications mentioned in this specification are indicative of the level of skill of those skilled in the art to which this invention pertains. All publications and patent applications cited are herein incorporated by reference to the same extent as if each individual publication or patent application was specifically and individually indicated to be incorporated by reference.

The invention now being fully described, it will be apparent to one of ordinary skill in the art that many changes and modifications can be made thereto without departing from the spirit or scope of the appended claims.

**Claims**

1. A compound of the formula

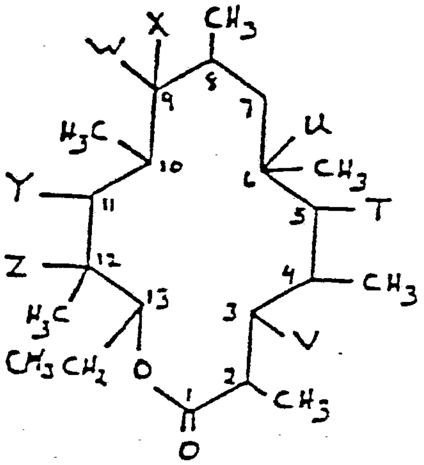

wherein:
T is OH or a pharmaceutically acceptable organic substituent attached to C5 through an oxygen;
V is OH or a pharmaceutically acceptable organic substituent attached to C3 through an oxygen;
U is hydrogen, hydroxy, or alkoxy or acyloxy of from 1 to 10 carbons, or U at C6 and an H at C7 are removed to form a double bond between C6 and C7, or U is taken together with X to define an ether bridge between C6 and C9;
Y is hydrogen, hydroxy, alkoxy or acyloxy of from 1 to 10 carbon atoms, a sulphate or sulfonate group bonded to C11 through an oxygen atom, $-OCH_2SO_2CH_3$, or $-OCH_2SOCH_3$; Y and H at C10 are removed to form a double bond between C10 and C11; or Y and W, when taken together with C9, C10, and C11 of the macrolide ring, represent a 5- to 7-membered heterocyclic ring; and
Z, X and W independently represent hydrogen, hydroxy, or acyloxy or alkoxy of 1 to 10 carbon atoms; with the provision that X and W taken together can represent $=O$ or $=S$; X can be taken together with U as defined above; W can be taken together with Y as defined above; or Z can be taken together with a hydrogen on C13 to represent a double bond.

2. A compound according to claim 1, wherein Y and W together with C9, C10 and C11 of the macrolide ring represent a 5- to 7-membered heterocyclic ring.

3. A compound according to claim 2, wherein said heterocyclic ring is a 6-membered 1,3-dioxa ring or a 6-membered 1,3-dioxa-2-bora ring, substituted with a phenyl group on the boron atom.

4. A compound according to claim 1, wherein said compound differs from the most closely related of erythromycin A or B by no more than 4 substituents represented by T, U, V, W, X, Y, and Z.

5. A compound according to claim 4, wherein T represents a sugar different from desosamine by 1 to 4 functional groups or V represents a sugar different from cladinose by 1 to 4 functional groups.

6. A compound of the formula:

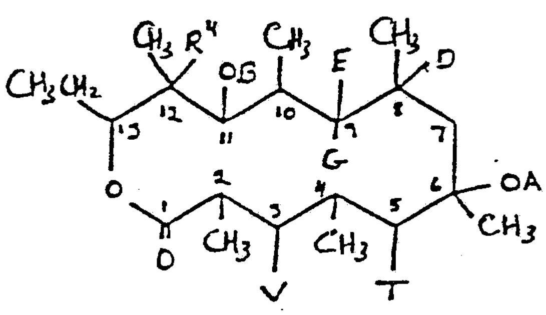

wherein:
T represents OH or a sugar residue;
V represents OH or a sugar residue;
A is hydrogen or acyloxy of from 1 to 10 carbons, or OA at C6 and an H at C7 are removed to form a double bond between C6 and C7, or A is taken together with G to define a bond, defining a vinyl ether bridge;
B is hydrogen, acyl, $CH_2SO_2CH_3$, or $CH_2SOCH_3$;
D is hydrogen or is taken together with E to define a double bond;
E is taken together with D to form a double bond or taken together with G to define an oximinoether where the oxygen is substituted with R; E and G taken together also can represent a diazo substituent or a diazo

substituent substituted with a pharmaceutically acceptable organic functional group;
R is a pharmaceutically acceptable organic substituent of from 1 to 20 carbon atoms; and
$R^4$ is hydrogen or hydroxyl.

7. A compound according to claim 1 or claim 6 wherein T is an amino-substituted sugar.

8. A compound according to claim 1 or claim 6 wherein T is 2-methyl-4-($R^1R^2N$)-5-$R^3$pyranyl-6-oxy, 2-methyl-3,4-epoxy-5-$R^3$-pyranyl-6-oxy, or 2-methyl-3,4-dehydro-5-$R^3$-pyranyl-6-oxy, wherein $R^1$ represents hydrogen or methyl and $R^2$ represents hydrogen, methyl, benzyl, or benzyl substituted with a pharmaceutically acceptable organic substituent, and $R^3$ is hydroxy or a pharmacologically acceptable acyloxy of from 1 to 10 carbon atoms.

9. A compound according to claim 8, wherein T is 2-methyl-4-($R^1R^2N$)-5-$R^3$pyranyl-6-oxy with the provisos that when $R^2$ is a substituted benzyl group, the sustituents are selected from the group consisting of halo, $C_1$-$C_4$ alkyl or alkanoyl, hydroxy, carboxy, amino, alkyl or dialkyl amino, and nitro, and when $R^3$ is an acyloxy, $R^3$ is formyloxy, acetoxy, propionoxy, ethyl succinoxy, 3,4-dichlorobenzyloxy, or nicotinyloxy.

10. A compound according to claim 1 or claim 6 wherein V rperesents 2,4-dimethyl-3-$R^3$-4-methoxypyranyl-6-oxy wherein $R^3$ is hydroxy or a pharmaceutically acceptable acyloxy of from 1 to 10 carbon atoms.

11. A compound according to claim 10 wherein R is a substituted benzyl group and the substituents on R are halo, $C_1$-$C_4$ alkyl or alkanoyl, hydroxy, carboxy, amino, alkyl or dialkyl amino, or nitro.

12. A pharmaceutical composition for use in inhibiting virus replication, characterized by including as active ingredient at least one compound as claimed in any of the preceding claims.

13. A pharmaceutical composition for use in inhibiting virus replication, characterized by including as active ingredient at least one erythromycin 9-oximinoether, $\Delta^8$-9-desoxo-6,9-oxyerythromycin, or erythromycin having at least one pharmaceutically acceptable organic substituent selected from mactolide and amino sugar double bonds, epoxides, and N-oxides; heterocyclic rings fused to the macrolide ring through positions C9 and C11; and alkyl, acyl, thioalkyl, sulfate, and sulfonate derivatives of hydroxy groups present in natural erythromycins and erythronolides.

14. Use of a compound as claimed in any one of claims 1 to 11 for the manufacture of a medicament for treating viral diseases.

15. Use of a compound which is erythromycin 9-oximinoether, $\Delta^8$-9-desoxo-6,9-oxyerythromycin, or erythromycin having at least one pharmaceutically acceptable organic substituent selected from mactolide and amino sugar double bonds, epoxides, and N-oxides; heterocyclic rings fused to the macrolide ring through positions C9 and C11; and alkyl, acyl, thioalkyl, sulfate, and sulfonate derivatives of hydroxy groups present in natural erythromycins and erythronolides for the manufacture of a medicament for treating viral diseasess.

16. Use as claimed in claim 13 or 14 wherein the viral disease due to a retrovirus or a hepadna virus.

Claims for the following contracting states: ES, GR and AT

1. A method of making a pharmaceutical composition comprising admixing a compound of the formula

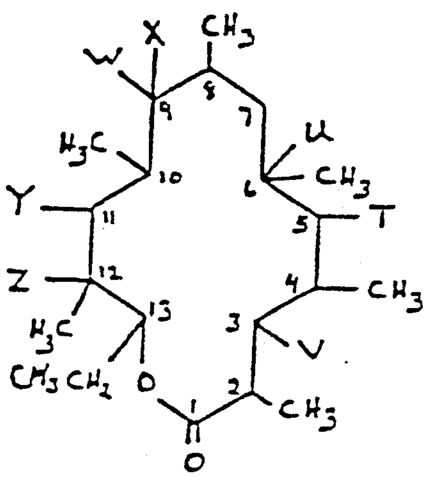

wherein:
T is OH or a pharmaceutically acceptable organic substituent attached to C5 through an oxygen;
V is OH or a pharmaceutically acceptable organic substituent attached to C3 through an oxygen;
U is hydrogen, hydroxy, or alkoxy or acyloxy of from 1 to 10 carbons, or U at C6 and an H at C7 are removed to form a double bond between C6 and C7, or U is taken together with X to define an ether bridge between C6 and C9;
Y is hydrogen, hydroxy, alkoxy or acyloxy of from 1 to 10 carbon atoms, a sulphate or sulfonate group bonded to C11 through an oxygen atom, $-OCH_2SO_2CH_3$, or $-OCH_2SOCH_3$; Y and H at C10 are removed to

form a double bond between C10 and C11; or Y and W, when taken together with C9, C10, and C11 of the macrolide ring, represent a 5- to 7-membered heterocyclic ring; and

Z, X and W independently represent hydrogen, hydroxy, or acyloxy or alkoxy of 1 to 10 carbon atoms; with the provision that X and W taken together can represent =O or =S; X can be taken together with U as defined above; W can be taken together with Y as defined above; or Z can be taken together with a hydrogen on C13 to represent a double bond.

2. A method with a pharmaceutically acceptable excipient and/or carrier according to claim 1, wherein Y and W together with C9, C10 and C11 of the macrolide ring represent a 5- to 7-membered heterocyclic ring.

3. A method according to claim 2, wherein said heterocyclic ring is a 6-membered 1,3-dioxa ring or a 6-membered 1,3-dioxa-2-bora ring, substituted with a phenyl group on the boron atom.

4. A method according to claim 1, wherein said compound differs from the most closely related of erythromycin A or B by no more than 4 substituents represented by T, U, V, W, X, Y, and Z.

5. A method according to claim 4, wherein T represents a sugar different from desosamine by 1 to 4 functional groups or V represents a sugar different from cladinose by 1 to 4 functional groups.

6. A method of making a pharmaceutical composition comprising admixing a compound of the formula:

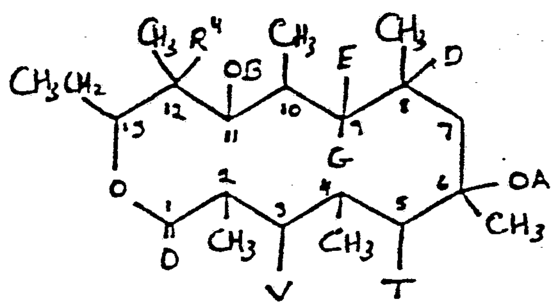

wherein:

T represents OH or a sugar residue;

V represents OH or a sugar residue;

A is hydrogen or acyloxy of from 1 to 10 carbons, or OA at C6 and an H at C7 are removed to form a double bond between C6 and C7, or A is taken together with G to define a bond, defining a vinyl ether bridge;

B is hydrogen, acyl, $CH_2SO_2CH_3$, or $CH_2SOCH_3$;

D is hydrogen or is taken together with E to define a double bond;

E is taken together with D to form a double bond or taken together with G to define an oximinoether where the oxygen is substituted with R; E and G taken together also can represent a diazo substituent or a diazo substituent substituted with a pharmaceutically acceptable organic functional group;

R is a pharmaceutically acceptable organic substituent of from 1 to 20 carbon atoms; and

$R^4$ is hydrogen or hydroxyl.

7. A method with a pharmaceutically acceptable excipient and/or carrier according to claim 1 or claim 6 wherein T is an amino-substituted sugar.

8. A method according to claim 1 or claim 6 wherein T is 2-methyl-4-($R^1R^2N$)-5-$R^3$pyranyl-6-oxy, 2-methyl-3,4-epoxy-5-$R^3$-pyranyl-6-oxy, or 2-methyl-3,4-dehydro-5-$R^3$-pyranyl-6-oxy, wherein $R^1$ represents hydrogen or methyl and $R^2$ represents hydrogen, methyl, benzyl, or benzyl substituted with a pharmaceutically acceptable organic substituent, and $R^3$ is hydroxy or a pharmaceutically acceptable acyloxy of from 1 to 10 carbon atoms.

9. A method according to claim 8, wherein T is 2-methyl-4-($R^1R^2N$)-5-$R^3$pyranyl-6-oxy with the provisos that when $R^2$ is a substituted benzyl group, the sustituents are selected from the group consisting of halo, $C_1$-$C_4$ alkyl or alkanoyl, hydroxy, carboxy, amino, alkyl or dialkyl amino, and nitro, and when $R^3$ is an acyloxy, $R^3$ is formyloxy, acetoxy, propionoxy, ethyl succinoxy, 3,4-dichlorobenzyloxy, or nicotinyloxy.

10. A method according to claim 1 or claim 6 wherein V rperesents 2,4-dimethyl-3-$R^3$-4-methoxypyranyl-6-oxy wherein $R^3$ is hydroxy or a pharmaceutically acceptable acyloxy of from 1 to 10 carbon atoms.

11. A method according to claim 10 wherein R is a substituted benzyl group and the substituents on R are halo, $C_1$-$C_4$ alkyl or alkanoyl, hydroxy, carboxy, amino, alkyl or dialkyl amino, or nitro.